# EUROPEAN PATENT APPLICATION

(11) **EP 0 743 045 A2**
(43) Date of publication of application: **20.11.1996**
(21) Application number: 96201055.9
(22) Date of filing: 19.04.1996
(51) Int. Cl.: A61B 17/064, A61B 17/70, A61F 2/44, A61B 17/68

(54) **Devices for osteosynthesis**

(30) Priority: 28.04.1995 RU 95107138; 27.09.1995 RU 95116522; 09.12.1995 RU 95121179
(71) Applicant: Gazzani, Romolo Igino, 15069 Serravalle Scrivia (Alessandria) (IT); Shaboldo, Oleg Pavlovich, St. Petersburg (RU)
(72) Inventor: Davydov, Evgeni Alexandrovich, Sestroretsk-1, St. Petersburg (RU); Davydov, Denis Evgenievich, Sestroretsk-1, St. Petersburg (RU); Shaboldo, Oleg Pavlovich, St. Petersburg (RU); Novoselov, Konstantin Anatolievich, St. Petersburg (RU); Zasulski, Philipp Yurievich, St. Petersburg (RU)
(74) Representative: Martegani, Franco

(57) **Abstract**

A device for osteosynthesis is of the kind prepared of material which exhibits form-memory effect, is made of wire and contains a working part and locking components. According to the invention the locking components are made as plunge legs; the working part consists of alternating arched components, with arch length exceeding 180°; and the adjacent components are placed to different sides of the line connecting their bending points to the plunge legs.

## Description

The invention is related to devices for osteosynthesis and can be used in traumatology, neurotraumatology, orthopedics, vertebrology, in case of traumas and surgical operations on the long tubular bones (including periathric regions), fractures of spine, vertebral dislocations, traumas and pathologies of other skeleton bones.

A number of plunge constructions have been suggested for use in surgery for the fixation of bone fragments: different kinds of plates, screws, wires, nails, etc.. See Williams D.Ph., Rouf R. Implantants in surgery. (in Russian), M; Medicine, 1978, pp. 409-450.

The majority of plunge constructions (especially those with compession mechanism) all share one main disadvantage: the effect of compression lasts only for several days, afterwards device is turning into a sort of "spacer", which leads to the instability of bone fragments that is, to violations of the reparative regeneration. See Devyatov A.A. Transosseous osteosynthesis. Kishinev; Shtinica, 1990, p. 314.

To avoid such complications, devices of a material displaying form-memory effect are being applied succesfully ensuring constant bone fragments' compression for the whole therapy period. See Gunter V.E., Kostenko V.V., Mirgazinov M.Z. et al. The alloys with form memory in medicine. Tomsk; Tomsk University Ed., 1986, pp. 116-119; and Gunter V.E., Kostenko V.V., Mirgazinov M.Z. et al. The alloys with form memory in medicine. Tomsk; Tomsk University Ed., 1986, pp. 68-73.

Depending on type of the fracture (pathology) it is often necessary, aside from firm fixation of bone fragments, to ensure required compressing force or to substitute bone fragment with a prosthesis keeping the function of the missing fragment. In these cases the form of the implantant made of material with form-memory effect is crucial.

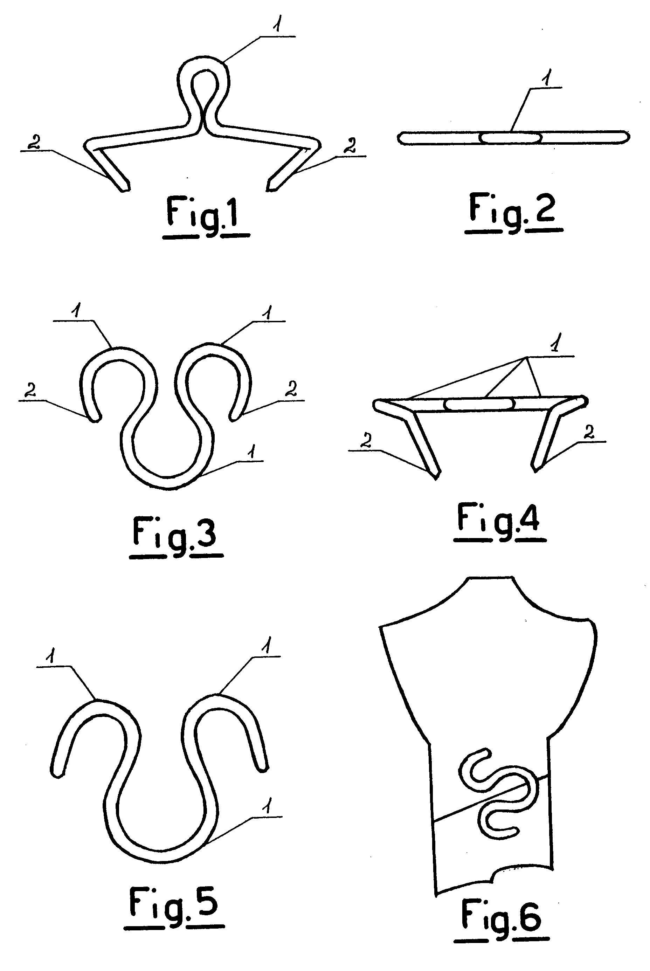

From the devices of alloys with form-memory effect taken into medical practice the most convenient are wire constructions of titanium nikelide. One of these devices (figures 1, 2)is made in the shape of a clip and contains two tapered legs (2) and working part (1), the last consisting of one or several arched components (rings), positioned in the same or orthogonal planes relative to the legs. See Gunter V.E., Etin V.I., Monasevich L.I. et al. Form-memory effects and their application in medicine. Novosibirsk; Nauka, Sib. Branch., 1992, pp.502-518. Prototype.

Besides, the middle part of the device is made arch-shaped. To decrease mass and overall dimensions of the device keeping unchanged the developed force it is made of material with low temperature values of the phase transition.

This device of the prior art has following disadvantages:
1. The developed force when clamping long tubular bone fragments is insufficient.
2. Inapplicable for treatment of the periathric fractures of long tubular bone fractures and joint pathologies (e.g., correcting osteotomy).
3. When shaping the device in the cooled state to a form convenient for the installation it is necessary to deform its components separately, thus complicating surgical operation and increasing its duration.
4. When using a material with low temperature values of the phase transition in devices with small working rings' diameter, destruction of the devices in the postoperative period is possible due to the overstrain of the material.
5. The device can be used in surgical operations on the spine with vertebral fractures and dislocation fractures. However, this may make vertebrae destruction possible because of the locking component (hook) action, which leads to the complications in the postoperative period. It also eliminates employment of the device in a number of cases, concerning fractures of different vertebral body parts (e.g., spinous process).
6. This device as well as related to it by the technical essence and attained results are made of alloys on the basis of titanium nikelide with low temperatures of the phase transition (T= -30...+35°C). These devices' application in neurosurgery may lead to the form-memory effect occuring during the installation, which aside from the difficulties for the surgeon presents danger for the patient's life (injuries of the open spinal marrow regions are possible). Liquid nitrogen or chloroethyl utilization for the device cooling also complicates the course of the operation.
7. The device is practically inapplicable in surgical operations on the spine connected with intervertebral disk removal, for it does not ensure recovery of the normal (initial) anatomic alignment of the vertebrae and the spine axis.

The general object of the present invention is to obviate the aforesaid disadvantages of the prior art.

This object is attained by devices for osteosynthesis having the features exposed in the annexed claims.

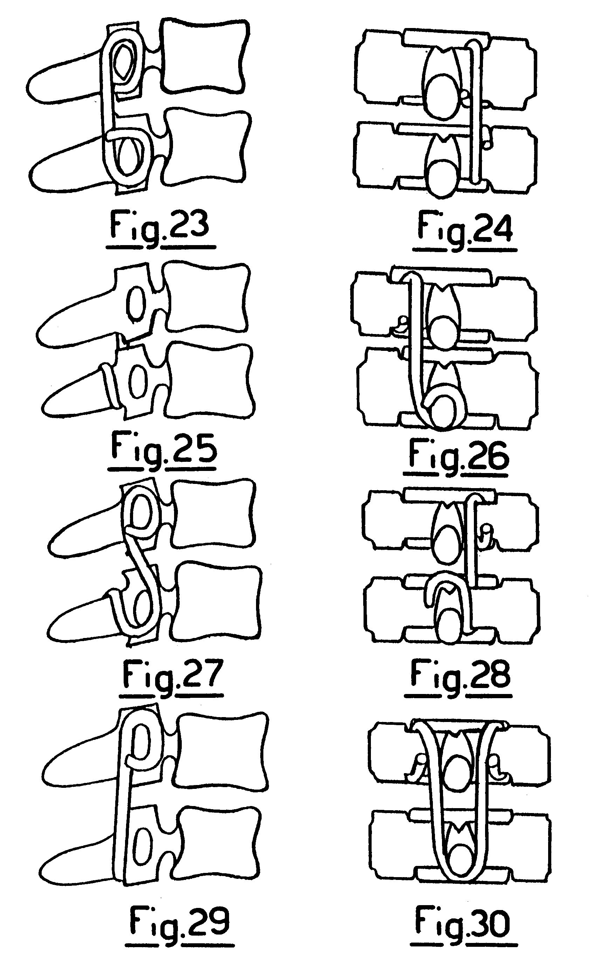

Figures 3-36 show embodiments of the invention.

Suggested solution according to the present invention is directed towards creating a set of the wire devices prepared from the material with form-memory effect and includes several versions.

Each of the proposed devices can be used separately during fracture therapy and surgical operations on the long tubular bones (including periarthric regions), in therapy of the spine fractures, vertebral dislocations, as well as in combinations with one another in course of more complicated operations.

To provide compression osteosynthesis, the device (of material, exhibiting form-memory effect) made of the wire and containing working part and locking components, is used. The locking components are done as plunge legs while the working part consists of alternating arched components (with the arch length exceeding 180°). The adjacent arched components are located at different sides of the line which connects their bending points with the plunge legs (compression line).

A version of the proposed implantant is represented on fig.3, 4 (working state, top view, side view): 1- arched components, 2- tapered plunge legs. Fig.5 shows the device in its deformed state after cooling.

Placing the adjacent arched components (with the arch length exceeding 180°) at different sides of the line which connects their bending points with the legs significantly facilitates device deformation in the cooled state. It also decreases overall device dimensions with given power parameters' values, increases power characteristics with given geometrical parameters (e.g., up to 116N for the proposed device from 80N for the prototype) and allows to avoid shifts of the bone fragments in course of the operation when the device restores its shape. Decrease of the device sizes also lowers traumaticity of the surgical intervention.

The device was tested by surgical operations on the long tubular bones for the fixation of bone fragments.

The device installation layout is represented on fig.6.

Employment of the device allows to avoid the postoperative immobilization and to begin early exercises of the joints adjacent to the osteosynthesis site.

When dealing with the oblique and comminuted periarthric fractures of the long tubular bones the compressing component developed by the working part of the device (according to the invention) significantly decreases due to the considerable change of the bone relief in its upper and lower thirds and, consequently, unparallel bone and device axes. Besides, the device does not adjoin the bone surface tightly.

Constant and high compression levels in the wire device for osteosynthesis (which is prepared from material with form-memory effect and contains working part and locking components) and rather tight adjoining of the later to the bone surface are required for satisfactory results on the oblique and comminuted periartric fractures of the long tubular bones. To meet this requirements, the locking components are made as plunge legs, one of which is supplied with an arched element made into the shape of the adjacent bone surface. In addition, the length of the leg with the arched element is 1,5-2,0 times greater than that of another leg, while the working part is 1,5-2,0 times shorter than the distance between the plunge legs.

Tight adjoining of device to the bone surface is achieved by:
i) making the working component 1,5-2,0 times shorter than device as a whole;
ii) arched component with the form of a curved metaphysis surface (adjacent bone surface region);
iii) encreasing the length of the straight leg plunged into the metaphysis 1,5-2,0 times deeper compared to the other one, plunged into diaphysis.

In addition, the working component's longitudinal axis is parallel to the bone axis, which allows to avoid the "parasite" shifting component of the force, appearing in the device when it restores its form as a result of heating. The working part (with the legs made as described above) not only can be formed by the arched components but may have any shape depending on specific osteosynthesis. Nevertheless, the most preferable working part is the one that consists of alternating arched components (the arch length is more than 180°) located at both sides of the line connecting the plunge legs with bending points of arched components. Besides, the device with such compressing working element can be deformed easily in the cooled state that facilitates putting it into a wound.

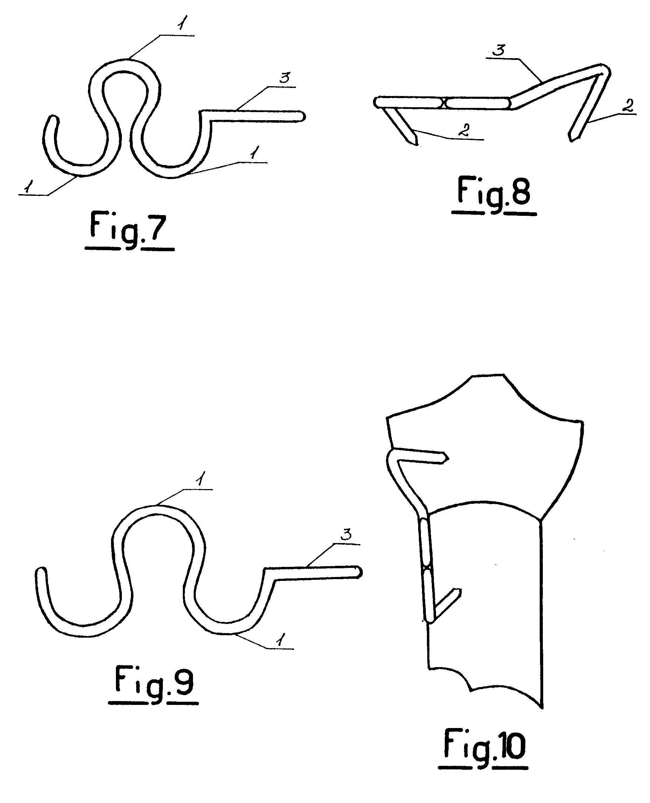

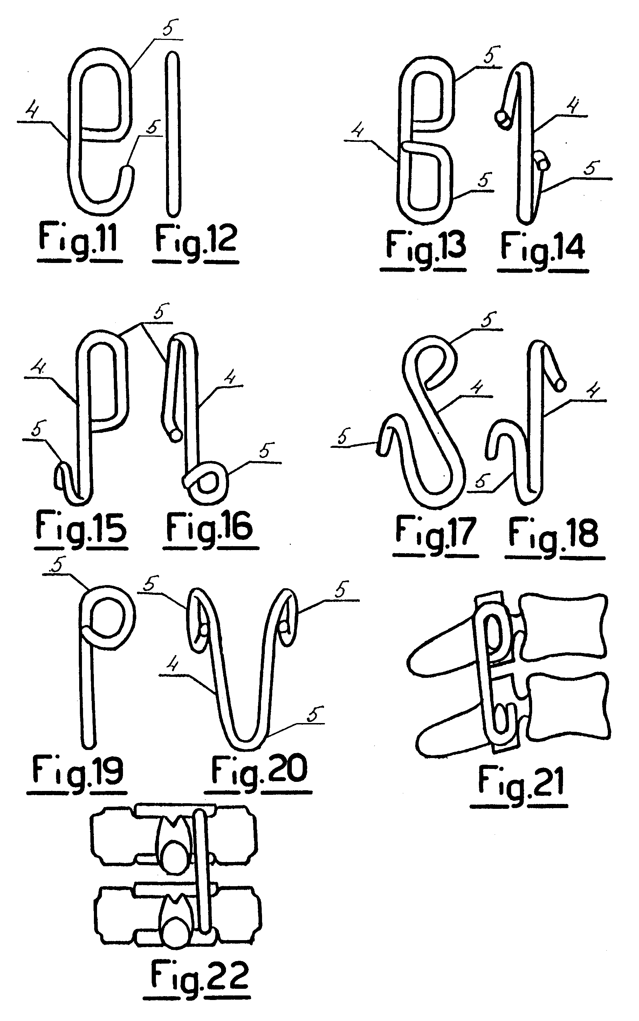

The essence of the invention is explained by diagrams on fig.7 (top view, in the working state), fig.8 (side view), fig.9 (deformed after cooling), fig. 10 (working state - set up on a bone).

The device includes: working part consisting of the arched components 1 (arch length is more than 180°), the plunge legs 2 one of which is supplied with an arched element 3. Positioning the device on a bone so that the vector of the force developed in the device when it is regaining its form is directed parallel to the bone axis (to the compression line) is attained by:
i) compressing working part being 1,5-2,0 times shorter than the device as a whole,
ii) supplying one of the legs with an arched element with the form of adjoining bone region (of the metaphysis).

In addition, the maximal compression is attained when geometrical parameters of the device (length, width and the wire diameter) are minimal. However, this does not take place when the correlation of the working part's and the whole device's lengths is not correct. The relationship between the plunge legs' lengths is chosen so that the line connecting legs' ends is parallel to the compression (bone) axis. If the leg plunged into metaphysis is shorter than 1,5 lengths of the leg plunged into diaphysis, its fixation in the spongy bone won't be firm enough. If its length exceeds the double length of the shorter leg it is quite likely that the diaphysial plunge leg will slip out of its slot when the device restores its form.

Before installation the device is cooled down to +5...+10°C. The working part is deformed so that the distance between plunge legs increases at the expense of decrease of the working part curvature. The arched element 3 of the locking leg plunged into metaphysis is not deformed. The short leg is set into specially prepared slot in the diaphysial part of a long tubular bone while the long one - into the spongy metaphysial bone. When the device is heated (either spontaneously or by irrigating with warm physiological salt solution) the working part recovers its earlier set form and the plunge legs are brought closer to each other. The legs are firmly clamped in the bone and the bone fragments - with one another. Meanwhile the device develops necessary compression.

The device was clinically tested with positive results in course of the valgoid subcondylar osteotomy of tibia in patients suffering from idiopathic arthrosis of the 2nd stage (by N.S.Kosinskaya) with varus crus deformity. The patients were treated by hinged osteotomy of tibia in the subcondylar zone. After simultaneous correction of the deformation the tibial bone fragments were clamped on both inner and outer surfaces with the proposed devices. Soft tissues were sutured without tension because of the device adaptation to the adjoining curved bone surface. The stable clamping of the bone fragments was achieved which allowed to bypass the external limb immobilization (on the 4th day) and to proceed to the early exercises of the knee joint.

To ensure reliable vertebrae fixation and holding in locomotory systems of all the spine sections, considering specificity of the traumas and pathologies of the individual organism with the simultaneous decrease of traumaticity and the spine surgical operation duration the following is proposed: in the clip-shaped wire device (made of the material, exhibiting form-memory effect) with the fixing components, at least one of the later represents a closed loop. The fixing components are placed in either one (in the same), parallel or perpendicular planes while the material should display form-memory effect in the temperature range of +30...+45°C.

Suggested device (versions) is represented on fig. 11-20 in the working state - side and back views. Fig. 21-30 show the device (its versions) being installed on the spine - side and back views.

The device consists of the wire clip 4 and the locking components 5 with the latter positioned either in one (fig. 11, 12), parallel (fig.13, 14, 19, 20) or perpendicular (fig.15-18) planes. The constructions shown on fig. 9-12 are convenient for eliminating of insignificant instability of the locomotory spine segment. These constructions can also be applied in more complicated cases in combinations with other versions of the device. They are easily installed on vertebral arches and spinous processes. Constructions (fig.15-18) are used on more critical traumas and pathologies (including development pathology), e.g., rotatory subluxations in the locomotary segment, caries of an arch of the underlying vertebrae, etc. These devices are installed on the upper vertebra arch and the lower vertebra spinous process (fig.25-28).

Constructions (fig. 19, 20) are employed on the bilateral subluxations in the locomotory segment. These are set up from both sides of the spine on the upper vertebra arch and put under the spinous process of the lower vertebra.

Making at least one of the locking components as a closed loop is aimed to ensure reliable clamping of the device (on either vertebral archs or spinous processes). Positioning locking components in the same, parallel or perpendicular planes allows the realization of the firm vertebrae clamping with the whole variety of the spine traumas and pathologies.

The design of the device and the installation procedure are fairly easy and it does not take very much time for the surgeon to put it in (1-2 minutes is enough). This is the reason to use a material (exhibiting form-memory effect) with the phase transition top limit not greater than +45°C. At the meantime, the bottom limit has to be +30°C or higher to prevent premature spontaneous operating of the device. Indicated temperature values prevent recovery of-the previously set form by the device when heated to the temperature of the operating room. The device made from such material does not require specific conditions for cooling and can be shaped al + 10...+20°C.

The device is applied as follows: the inferior access to the required spine section is perfomed and depending on the trauma or pathology type needed version of the device (several versions at a time can also be used) is chosen and employed separately or in combination with other known devices prepared from materials with form-memory effect. The fixation is perfomed on either arches or arches with spinous processes (the components of the device are preliminary cooled down to +10...+20°C and shaped to the form convenient to put it in). After the installation the device is heated up by warm physiological salt solution, locking components recover their initial form and firmly clamp the spine.

Employment of the proposed device allows to achieve reliable postoperative stability by considering the specificity of traumas, pathologies and the individual organism structure of a patient as well as to completely recover the function of the injured spine segment, decrease traumaticity and duration (by up to 20-30%) of the surgical operation.

For obtaining the best results of the surgical treatment of the spine it is necessary as soon as possible to eliminate the compression of the spinal marrow and to recover normal (initial) anatomical correlation of the vertebrae and the spine axis. In particular, operations on the vertebral unstable fractures or dislocation fractures makes necessary either:
i) intervertebral disk removal with the following firm clamping to achieve primary postoperative stability (until the osteofibrous block formation); or
ii) consolidation on the level of the injured locomotory spine segment.

Indicated therapy methods are put into the practice by making device in the shape of a single-coil helix, which is made of the material with formmemory effect at +30...+45°C temperature range. At its ends the helix has tapered legs, directed to the opposite sides of the line parallel to the helix axis. This device serves as a fixator, being, as a matter of fact, an endoprosthesis of the intervertebral disk. It provides the decrease of traumaticity and surgical operation duration, prevents vertebrae from shifting, eliminates the danger of the spinal marrow compression and ensures maximal recovery of the anatomical spine structure and its functioning in the early postoperative period.

The essense of the invention is shown on the diagrams:

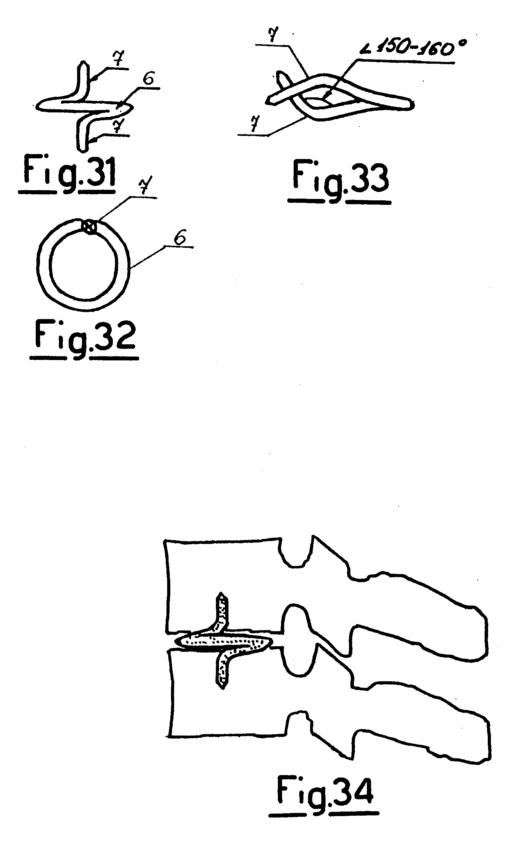

fig. 31 - the device at its working state (side view);
fig. 32 - top view; fig. 33 - the device after deformation; fig. 34 - how-to-use scheme.

The device is made out of a material exhibiting form-memory effect at +30...+45°C, e.g., of the alloy based on titanium nikelide. Some operations in neurosurgery (e.g., surgurical operations on the vertebral column) require considerable time for the implantant installation and the premature form recovery by the device is anadmissible in this case. This demand narrows the temperature range for the form-memory effect. The device described at the beginning of this paragraph meets this requirement and is applicable in this case. Narrowing the temperature range of the form-memory effect provides (by serial endoprosthesis production) obtaining of the appliances with close levels of the physicomechanical characteristics both at the human body and room temperatures. To transfer the device to the low-temperature state and impart to it a form convenient for the installation, chloroethyl cooling is not required. It is sufficient to cool it to +5...+15°C by irrigating with cooled sterile solution.

The material with indicated parameters does not begin to exhibit form-memory effect and to restore the given form until heated up to 30°C. This feature eliminates endoprosthesis' operation at room temperature in hand of the medical personell.

Spiral form of the working part 6 allows the device to be shaped easily in the cooled state. When it deforms, the helix coil's edges 6 are brought closer to each other, while the legs 7 embed into vertebral body forming an angle between the leg and the helix turn (the angle is about 150...160°). After the installation as a result of heating by warm physiological salt solution, tapered ends spontaneously plunge into vertebral body. This, together with the legs positioning- in their working state on one line (which is parallel to the helix turn axis that is, to the spine) leads to the endoprosthesis set evenly, without skews, thus preventing vertebrae shifts and holding them.

The endoprothesis is applied as follows:

The regular anterior access to the neck region or inferior lateral access to the chest and lumbar regions of the spine is perfomed. The intervertebral disk is removed. Vertical punctures (from the limbial side) are made through the adjacent vertebral bodies. The punctures serve as guides for the endoprothesis legs.

The endoprosthesis is cooled to +5...+15°C with the sterile (e.g., physiological) solution and is shaped into a form convenient for being inserted into the space resulting from the invertebral disk removal.

The legs 7 of the helix coil 6 are brought towards one another and turned so as to decrease the endoprosthesis vertical size (fig.33). The endoprosthesis is set to the space between the vertebrae by the forceps so that the legs fit into the prepared slots. The following irrigation with warm sterile solution (about 50°C) causes the deformation of the device, it restores its preliminary set form which ensures the stabilization of the injured spine segment. Simplicity of the construction and the employment convenience allow to decrease significantly traumaticity and the surgica] operation duration. Endoprosthesis provides reliable vertebrae clamping and primary postoperative stabilization (use of the soft flanges in the neck region and plaster jackets in the chest and lumbar regions is sufficient enough) as well as reduces treatment time. After the surgical operation with the use of the device a patient with no neurological deficiency is able-bodies in 1-2 months.

The device allows to recover the spine function in case of the intervertebral disk removal, traumas and illnesses and to prevent spondylolisthesis as well as axial spine deformity.

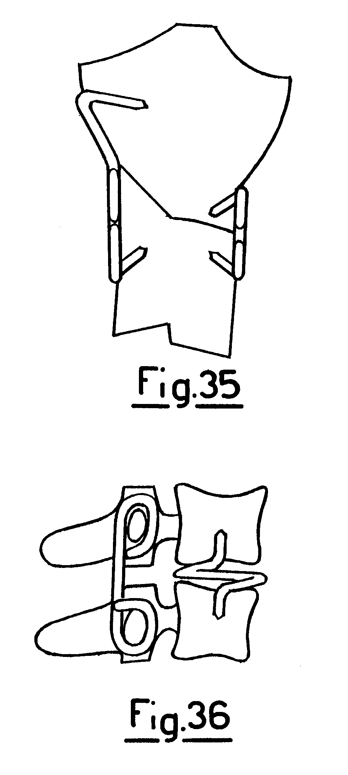

According to the invention the devices are applicable not only separately but in combination with one another. For example, in case of the compound fracture of the tibial upper third setting of several devices is needed (fig. 35). The spine trauma and intervertebral disk injury require vertebrae clamping and endoprosthesis setup (fig. 36).

The proposed device is prepared of wire (1,5-4 mm in diameter) of the material exhibiting form-memory effect (e.g., titanium nikelide with slight ni excess: 50,2-50,5 at. %). To provide form-memory effect in the required (depending on the version and purpose) temperature range, constructions are subjected to thermal and thermomechanical treatment by different known procedures. See Khachin V.M., Pushin V.G., Kondratyev V.V. Titanium nikelide: structure and properties. M; Nauka, 1992, pp .143- 146.

Application of the set of different versions of the device allows to obtain reliable clamping of the bone fragments and transplantants during operations on different skeleton sections in case of traumas and pathologies of various difficulty.

## Claims

1. The device for osteosynthesis is of the kind prepared of material which exhibits form-memory effect, is made of wire and contains a working part and locking components, and is characterized by the fact that, the locking components are made as plunge legs; the working part consists of alternating arched components, with arch length exceeding 180°; and the adjacent components are placed to different sides of the line connecting their bending points to the plunge legs.

2. The device according to claim 1 is characterised by the locking components made as the plunge legs one of which is supplied with an arched element made into the shape of adjoining bone surface.

3. The device according to claim 2 is characterised by the fact that the length of the leg supplied with the arched element is 1,5-2,0 times greater than that of the other leg.

4. The device according to claim 1 is characterised by the fact that the working part's length is 1,5-2,0 times shorter than the distance between the plunge legs.

5. The device according to claim 1 is characterised by the fact that the working part is formed by the arched components with the arch length exceeding 180° and the adjacent components located at different sides of the line which connects their bending points with the plunge legs.

6. The device according to claim 1 is characterised by the fact that the locking components are bent in either one, parallel or perpendicular planes with at least one of them being a closed loop.

7. The device according to claim 1 is characterised by the fact that the material exhibits form-memory effect in +30...+45°C temperature range.

8. The device according to claim 1 is characterised by the fact that the working part is done as a single-coil helix, while the locking components as tapered legs located at the helix's ends and directed along the line parallel to the helix axis.
